(19) Europäisches Patentamt
European Patent Office
Office européen des brevets

(11) Veröffentlichungsnummer: **0 154 806**
**A2**

# (12) EUROPÄISCHE PATENTANMELDUNG

(21) Anmeldenummer: **85101172.6**

(22) Anmeldetag: **05.02.85**

(51) Int. Cl.⁴: **C 07 D 303/14**, C 07 D 303/22, C 07 C 33/03, C 07 C 33/28, C 07 C 33/38, C 07 C 33/48, C 07 D 213/30, C 07 C 43/166, C 07 C 43/176, C 07 C 29/40, A 01 N 43/20

(30) Priorität: **15.02.84 DE 3405417**

(43) Veröffentlichungstag der Anmeldung: **18.09.85**
**Patentblatt 85/38**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Böckmann, Klaus, Dr., Andreas-Gryphius-Strasse 7, D-5000 Köln 80 (DE)**
Erfinder: **Lürssen, Klaus, Dr., August-Kierspel-Strasse 151, D-5060 Bergisch-Gladbach 2 (DE)**
Erfinder: **Hänssler, Gerd, Dr., Heymannstrasse 40, D-5090 Leverkusen 1 (DE)**
Erfinder: **Schmidt, Robert R. Dr., Im Waldwinkel 110, D-5060 Bergisch-Gladbach 2 (DE)**

## (54) Substituierte Oxirane.

(57) Neue substituierte Oxirane der Formel

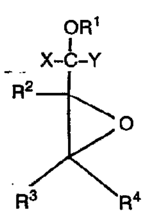

(I)

in welcher
X für gegebenenfalls substituiertes Alkyl, Cycloalkyl, gegebenenfalls substituiertes Phenyl, gegebenenfalls substituiertes Phenylalkyl, gegebenenfalls substituiertes Phenoxyalkyl oder gegebenenfalls substituiertes Phenalkoxyalkyl steht,
Y für Wasserstoff, gegebenenfalls substituiertes Alkyl, gegebenenfalls substituiertes Aryl, gegebenenfalls substituiertes Aralkyl, für gegebenenfalls substituiertes Cycloalkyl oder für Pyridyl steht, oder
X und Y gemeinsam für eine Alkylenkette mit 4 bis 6 Kohlenstoffatomen oder für die Reste der Formeln

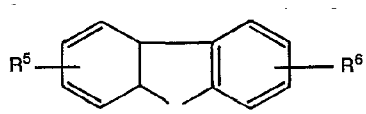

und

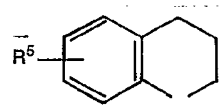

stehen, worin
$R^5$ und $R^6$ unabhängig voneinander für Wasserstoff, Halogen, Alkyl, Alkoxy, Alkylthio, Halogenalkyl oder Phenyl stehen,
$R^1$ für Wasserstoff, gegebenenfalls substituiertes Alkyl, Acyl, Alkenyl oder gegebenenfalls substituiertes Aralkyl steht und
$R^2$, $R^3$ und $R^4$ unabhängig voneinander für Wasserstoff oder Alkyl stehen,
mehrere Verfahren zur Herstellung der neuen Stoffe und deren Verwendung als Pflanzenbehandlungsmittel.

EP 0 154 806 A2

BAYER AKTIENGESELLSCHAFT
Konzernverwaltung RP
Patentabteilung

5090 Leverkusen, Bayerwerk
Dü/ABc
Ib

## Substituierte Oxirane

Die vorliegende Erfindung betrifft neue Oxirane, mehrere Verfahren zu deren Herstellung und ihre Verwendung als Pflanzenbehandlungsmittel.

Es ist bereits bekannt, daß bestimmte Dialkylamide der Bernsteinsäure pflanzenwachstumsregulierende Eigenschaften besitzen (vgl. Science 136, 391 (1962) und R. Wegler "Chemie der Pflanzenschutz- und Schädlingsbekämpfungsmittel" Band 4, Seiten 27 und 28, Springer-Verlag, Berlin-Heidelberg-New York, 1977). So kann zum Beispiel das Bernsteinsäure-mono-N-dimethyl-hydrazid zur Regulierung des Pflanzenwachstums eingesetzt werden. Die Wirkung dieses Stoffes ist jedoch vor allem bei niedrigen Aufwandmengen nicht immer befriedigend.

Ferner ist bereits bekannt geworden, daß Zink-ethylen-1,2-Bisdithiocarbamidat ein gutes Mittel zur Bekämpfung von pilzlichen Pflanzenkrankheiten ist (vgl. Phytopathology 33, 1113 (1963)). Jedoch ist dessen Einsatz

nur beschränkt möglich, da es insbesondere bei niedrigen Aufwandmengen und -konzentrationen nicht immer befriedigend wirksam ist.

Es wurden nun neue substituierte Oxirane der Formel

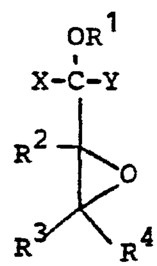

(I)

in welcher

X für gegebenenfalls substituiertes Alkyl, Cycloalkyl, gegebenenfalls substituiertes Phenyl, gegebenenfalls substituiertes Phenylalkyl, gegebenenfalls substituiertes Phenoxyalkyl oder gegebenenfalls substituiertes Phenalkoxyalkyl steht,

Y für Wasserstoff, gegebenenfalls substituiertes Alkyl, gegebenenfalls substituiertes Aryl, gegebenenfalls substituiertes Aralkyl, für gegebenenfalls substituiertes Cycloalkyl oder für Pyridyl steht, oder

X und Y gemeinsam für eine Alkylenkette mit 4 bis 6 Kohlenstoffatomen oder für die Reste der Formeln

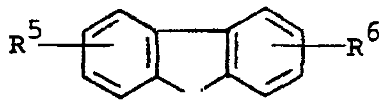

und

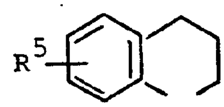

stehen, worin

$R^5$ und $R^6$ unabhängig voneinander für Wasserstoff, Halogen, Alkyl, Alkoxy, Alkylthio, Halogenalkyl oder Phenyl stehen,

$R^1$ für Wasserstoff, gegebenenfalls substituiertes Alkyl, Acyl, Alkenyl oder gegebenenfalls substituiertes Aralkyl steht und

$R^2$, $R^3$ und $R^4$ unabhängig voneinander für Wasserstoff oder Alkyl stehen,

gefunden.

Diejenigen erfindungsgemäßen Stoffe, die ein oder mehrere unsymmetrisch substituierte Kohlenstoffatome enthalten, können in Form von Racematen oder auch in Form von Diastereomerengemischen vorliegen. Die Erfindung betrifft sowohl die Isomerengemische als auch die einzelnen Isomeren.

Weiterhin wurde gefunden, daß man substituierte Oxirane der Formel (I) erhält, wenn man

a) substituierte Alkene der Formel

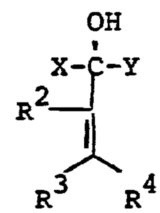

(II)

in welcher

X, Y, $R^2$, $R^3$ und $R^4$ die oben angegebene Bedeutung haben,

mit Epoxidierungsmitteln gegebenenfalls in Gegenwart eines inerten organischen Verdünnungsmittels umsetzt,

oder

b) substituierte Alkene der Formel

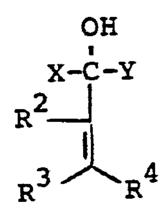

(II)

in welcher

X, Y, $R^2$, $R^3$ und $R^4$ die oben angegebene Bedeutungen haben,

zunächst mit Verbindungen der Formel

Z - R (III)

in welcher

R für gegebenenfalls substituiertes Alkyl, Acyl, Alkenyl oder gegebenenfalls substituiertes Aralkyl steht und

Z für Halogen, Tosylat oder Mesylat steht,

gegebenenfalls in Gegenwart eines Säurebindemittels und gegebenenfalls in Gegenwart eines inerten organischen Verdünnungsmittels umsetzt und die dabei anfallenden Verbindungen der Formel

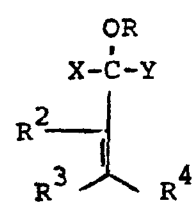

(IV)

in welcher

X, Y, R, $R^2$, $R^3$ und $R^4$ die oben angegebene Bedeutung haben,

dann mit Epoxidierungsmitteln gegebenenfalls in Gegenwart eines inerten organischen Verdünnungsmittels umsetzt.

Schließlich wurde gefunden, daß sich die substituierten Oxirane der Formel (I) sehr gut zur Behandlung von Pflanzen eignen. Sie zeichnen sich durch vorzügliche pflanzenwuchsregulierende, fungizide und herbizide Eigenschaften aus.

Überraschenderweise besitzen die erfindungsgemäßen substituierten Oxirane der Formel (I) eine deutlich bessere pflanzenwuchsregulierende Wirksamkeit als das bekannte Bernsteinsäure-mono-N-dimethylhydrazid, welches ein anerkannt gut wirksamer Stoff gleicher Wirkungsart ist. Außerdem zeigen die erfindungsgemäßen Verbindungen überraschenderweise auch bessere fungizide Eigenschaften als das aus dem Stand der Technik bekannte Zink-ethylen-1,2-bisdithiocarbamidat, welches eine wirkungsmäßig naheliegende Verbindung ist.

Die erfindungsgemäßen Stoffe sind durch die Formel (I) allgemein definiert. In dieser Formel steht X vorzugsweise für gegebenenfalls durch Halogen, Alkoxy mit 1 bis 6 Kohlenstoffatomen und/oder Alkylthio mit 1 bis 6 Kohlenstoffatomen substituiertes Alkyl mit 1 bis 12 Kohlenstoffatomen, Cycloalkyl mit 3 bis 8 Kohlenstoffatomen oder für den Rest der Formel

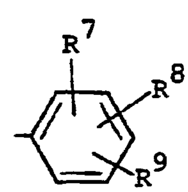 ,

in welcher $R^7$, $R^8$ und $R^9$ unabhängig voneinander für Wasserstoff, Halogen, Alkyl, Alkoxy, Alkylthio, Halogenalkyl oder Phenyl stehen.

Weiterhin steht X vorzugsweise für gegebenenfalls durch Halogen und/oder Alkyl substituiertes Phenylalkyl mit 1 bis 4 Kohlenstoffatomen im Alkylteil, für gegebenenfalls durch Halogen und/oder Alkyl substituiertes Phenoxyalkyl mit 1 bis 4 Kohlenstoffatomen im Alkylteil oder für gegebenenfalls durch Halogen und/oder Alkyl substituiertes Phenalkoxyalkyl mit 1 bis 4 Kohlenstoffatomen in der Alkoxygruppe und 1 bis 4 Kohlenstoffatomen im Alkylteil.

Y steht vorzugsweise für Wasserstoff, gegebenenfalls durch Halogen, Alkoxy mit 1 bis 6 Kohlenstoffatomen und/oder Alkylthio mit 1 bis 6 Kohlenstoffatomen substituiertes Alkyl mit 1 bis 12 Kohlenstoffatomen, gegebenenfalls durch Halogen, Alkyl, Alkoxy, Alkylthio, Halogenalkyl und/oder Phenyl substituiertes Aryl mit 6 bis 10 Kohlenstoffatomen, für gegebenenfalls durch Halogen, Alkyl, Alkoxy, Alkylthio, Halogenalkyl und/oder Phenyl substituiertes Aralkyl mit 6 bis 10 Kohlenstoffatomen im Arylteil und 1 bis 4 Kohlenstoffatomen im Alkylteil, für gegebenenfalls durch Alkyl mit 1 bis 6 Kohlenstoffatomen substituiertes Cycloalkyl mit 3 bis 8 Kohlenstoffatomen oder für Pyridyl.

Außerdem stehen X und Y gemeinsam für eine Alkylenkette mit vorzugsweise 4 oder 5 Kohlenstoffatomen oder für die Reste der Formeln

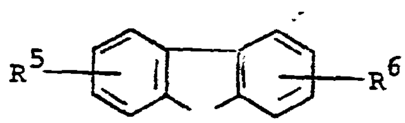 und 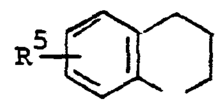 ,

in welchen

$R^5$ und $R^6$ unabhängig voneinander vorzugsweise für Wasserstoff, Fluor, Chlor, Brom, Alkyl mit 1 bis 6 Kohlenstoffatomen, Alkoxy mit 1 bis 6 Kohlenstoffatomen, Alkylthio mit 1 bis 6 Kohlenstoffatomen, Halogenalkyl mit 1 bis 6 Kohlenstoffatomen und 1 bis 5 Halogenatomen oder Phenyl stehen.

$R^1$ steht vorzugsweise für Wasserstoff, gegebenenfalls durch Fluor, Chlor, Brom, Alkoxy mit 1 bis 6 Kohlenstoffatomen und/oder Alkylthio mit 1 bis 6 Kohlenstoffatomen substituiertes Alkyl mit 1 bis 6 Kohlenstoffatomen, Alkylcarbonyl mit 1 bis 6 Kohlenstoffatomen im Alkylteil, Phenylcarbonyl, für Alkenyl mit 2 bis 8 Kohlenstoffatomen oder für gegebenenfalls durch Halogen und/oder Alkyl substituiertes Aralkyl mit 6 bis 10 Kohlenstoffatomen im Arylteil und 1 bis 4 Kohlenstoffatomen im Alkylteil.

$R^2$, $R^3$ und $R^4$ stehen unabhängig voneinander vorzugsweise für Wasserstoff oder Alkyl mit 1 bis 4 Kohlenstoffatomen.

Besonders bevorzugt sind diejenigen Stoffe der Formel (I), in denen

X für gegebenenfalls durch Fluor, Chlor, Brom, Alkoxy mit 1 bis 4 Kohlenstoffatomen und/oder Alkylthio mit 1 bis 4 Kohlenstoffatomen substituiertes Alkyl mit 1 bis 6 Kohlenstoffatomen, Cycloalkyl mit 3 bis 7 Kohlenstoffatomen oder für den Rest der Formel

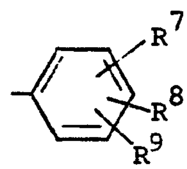

steht, worin,

$R^7$, $R^8$ und $R^9$ unabhängig voneinander für Wasserstoff, Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 3 Fluor- und/oder Chloratomen oder für Phenyl stehen,

oder

X für gegebenenfalls durch Fluor, Chlor, Brom und/oder Alkyl mit 1 bis 4 Kohlenstoffatomen substituiertes Phenylalkyl mit 1 oder 2 Kohlenstoffatomen im Alkylteil, für gegebenenfalls durch Fluor, Chlor, Brom und/oder Alkyl mit 1 bis 4 Kohlenstoffatomen substituiertes Phenoxyalkyl mit 1 oder 2 Kohlenstoffatomen

substituiertes Phenoxyalkyl mit 1 oder 2 Kohlenstoffatomen im Alkylteil oder für gegebenenfalls durch Fluor, Chlor, Brom und/oder Alkyl mit 1 bis 4 Kohlenstoffatomen substituiertes Phenalkoxyalkyl mit 1 oder 2 Kohlenstoffatomen im Alkoxyteil und 1 oder 2 Kohlenstoffatomen im Alkylteil steht,

Y für Wasserstoff, gegebenenfalls durch Fluor, Chlor, Brom, Alkoxy mit 1 bis 4 Kohlenstoffatomen und/oder Alkylthio mit 1 bis 4 Kohlenstoffatomen substituiertes Alkyl mit 1 bis 6 Kohlenstoffatomen, für gegebenenfalls durch Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 3 Fluor- und/oder Chloratomen und/oder Phenyl substituiertes Phenyl oder Naphthyl, für gegebenenfalls durch Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 3 Fluor- und/oder Chloratomen und/oder Phenyl substituiertes Phenylalkyl oder Naphthylalkyl mit jeweils 1 oder 2 Kohlenstoffatomen im Alkylteil, für gegebenenfalls durch Alkyl mit 1 bis 4 Kohlenstoffatomen, insbesondere durch Methyl und/oder Ethyl, substituiertes Cycloalkyl mit 3 bis 8 Kohlenstoffatomen oder für Pyridyl steht, oder

X und Y gemeinsam für eine Alkylkette mit 4 oder 5 Kohlenstoffatomen oder für die Reste der Formeln

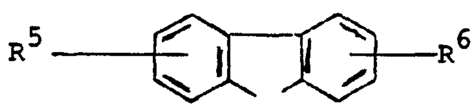

und

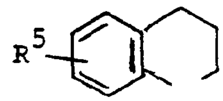

stehen, worin

$R^5$ und $R^6$ unabhängig voneinander besonders bevorzugt für Wasserstoff, Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 3 Fluor- und/oder Chloratomen oder für Phenyl stehen,

$R^1$ für Wasserstoff, gegebenenfalls durch Fluor, Chlor, Brom, Alkoxy mit 1 bis 4 Kohlenstoffatomen und/oder Alkylthio mit 1 bis 4 Kohlenstoffatomen substituiertes Alkyl mit 1 bis 4 Kohlenstoffatomen, Alkylcarbonyl mit 1 bis 4 Kohlenstoffatomen im Alkylteil, Phenylcarbonyl, für Alkenyl mit 2 bis 6 Kohlenstoffatomen oder für gegebenenfalls durch Fluor, Chlor, Brom und/oder Alkyl mit 1 bis 4 Kohlenstoffatomen substituiertes Phenylalkyl mit 1 oder 2 Kohlenstoffatomen im Alkylteil steht und

$R^2$, $R^3$ und $R^4$ unabhängig voneinander für Wasserstoff, Methyl, Ethyl oder Propyl stehen.

Als Beispiele für erfindungsgemäße Stoffe seien die in der folgenden Tabelle aufgeführten Verbindungen genannt:

Tabelle 1

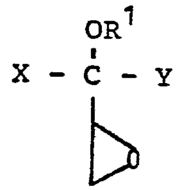

(Ia)

| X | Y | $R^1$ |
|---|---|---|
| 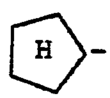 | 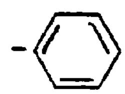 | H |
| 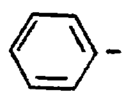 | 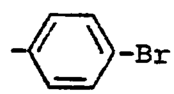 | H |
| $CH_3$ | 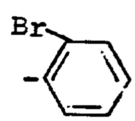 | H |
| $CH_3$ | 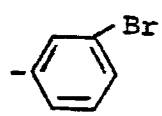 | H |
| $CH_3$ | 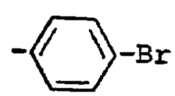 | H |
| $CH_3$ | 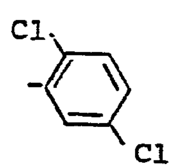 | H |
| $CH_3$ | 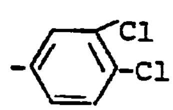 | H |

Tabelle 1 (Fortsetzung)

| X | Y | $R^1$ |
|---|---|---|
| $CH_3$ | 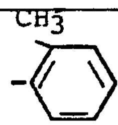 | H |
| $CH_3$ | 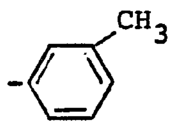 | H |
| $CH_3$ | 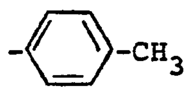 | H |
| $CH_3$ | 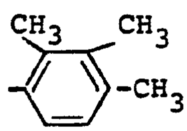 | H |
| 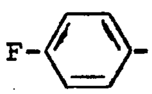 | 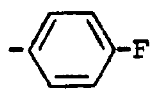 | H |
| 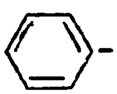 | 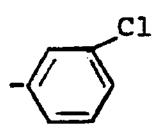 | H |
| $C_2H_5$ | 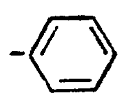 | H |
| $C_2H_5$ | 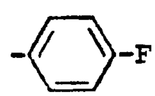 | H |
| $CH_2Cl$ | 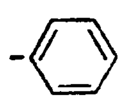 | H |

Tabelle 1 (Fortsetzung)

| X | Y | $R^1$ |
| --- | --- | --- |
| 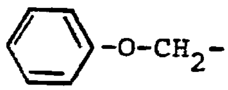 | 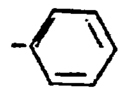 | H |
| 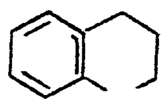 | | H |
| 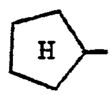 | 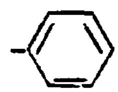 | $CH_3$ |
| 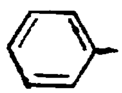 | 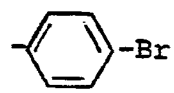 | $CH_3$ |
| $CH_3$ | 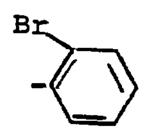 | $CH_3$ |
| $CH_3$ | 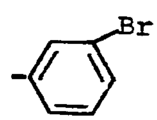 | $CH_3$ |
| $CH_3$ | 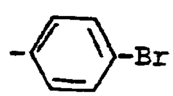 | $CH_3$ |
| $CH_3$ | 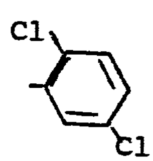 | $CH_3$ |
| $CH_3$ | 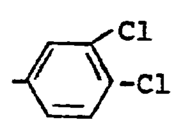 | $CH_3$ |

Tabelle 1 (Fortsetzung)

| X | Y | $R^1$ |
|---|---|---|
| $CH_3$ | 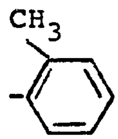 | $CH_3$ |
| $CH_3$ | 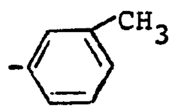 | $CH_3$ |
| $CH_3$ | 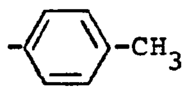 | $CH_3$ |
| $CH_3$ | 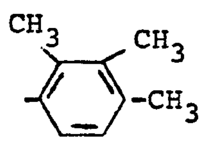 | $CH_3$ |
| 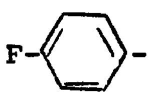 | 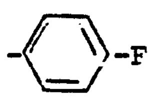 | $CH_3$ |
| 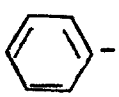 | 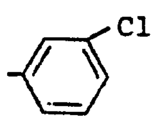 | $CH_3$ |
| $C_2H_5$ | 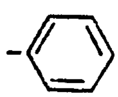 | $CH_3$ |
| $C_2H_5$ | 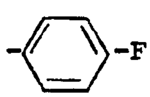 | $CH_3$ |

Tabelle 1 (Fortsetzung)

| X | Y | $R^1$ |
|---|---|---|
| $CH_2Cl$ | 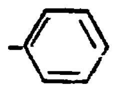 | $CH_3$ |
| 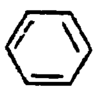$-O-CH_2-$ | 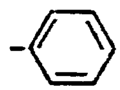 | $CH_3$ |
| 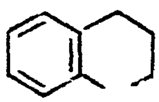 | | $CH_3$ |

Verwendet man 1,1-Diphenyl-prop-2-en-1-ol und 3-Chlorperbenzoesäure als Ausgangsstoffe, so kann der Verlauf des erfindungsgemäßen Verfahrens (a) durch das folgende Reaktionsschema wiedergegeben werden:

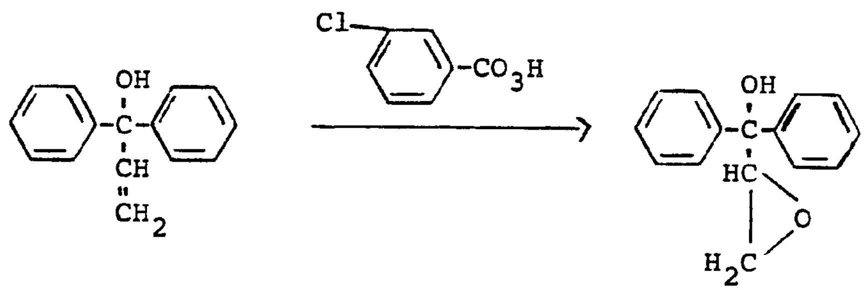

Verwendet man 1,1-Diphenyl-prop-2-en-1-ol und Methyliodid als Ausgangsstoffe und 3-Chlorperbenzoesäure als Reaktionskomponente, so kann der Verlauf des erfindungsgemäßen Verfahrens (b) durch das folgende Reaktionsschema wiedergegeben werden:

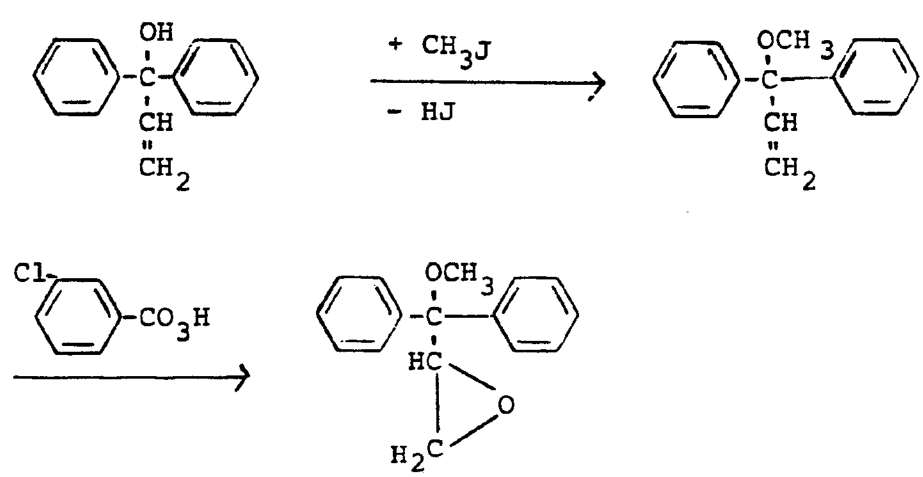

Die bei dem erfindungsgemäßen Verfahren (a) als Ausgangsstoffe benötigten substituierten Alkene sind durch die Formel (II) allgemein definiert. In dieser Formel haben X, Y, $R^2$, $R^3$ und $R^4$ vorzugsweise diejenigen Bedeutungen, die bereits im Zusammenhang mit der Beschreibung der erfindungsgemäßen Stoffe der Formel (I) für diese Reste als bevorzugt genannt wurden.

Die substituierten Alkene der Formel (II) sind bisher noch nicht bekannt. Sie lassen sich herstellen, indem man Ketone oder Aldehyde der Formel

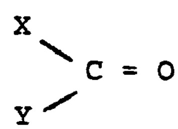

(V)

in welcher

X und Y die oben angegebene Bedeutung haben,

mit Grignard-Reagentien der Formel

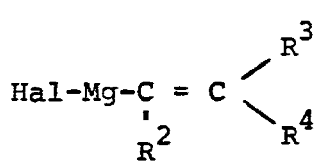

(VI)

in welcher

$R^2$, $R^3$ und $R^4$ die oben angegebene Bedeutung haben und

Hal für Brom oder Jod steht,

in Gegenwart eines inerten Verdünnungsmittels umsetzt.

Die bei der Herstellung der substituierten Alkene der Formel (II) nach dem obigen Verfahren als Ausgangsstoffe benötigten Ketone oder Aldehyde der Formeln (V) und (VI) sind bekannt oder lassen sich nach im Prinzip bekannten Verfahren in einfacher Weise synthetisieren.

Als Verdünnungsmittel kommen bei dem obigen Verfahren zur Herstellung der substituierten Alkene der Formel (II) alle für derartige Umsetzungen geeigneten inerten organischen Verdünnungsmittel in Betracht. Vorzugsweise verwendbar sind Ether, wie Diethylether, Dioxan oder Tetrahydrofuran.

Die Reaktionstemperaturen können bei der obigen Umsetzung zur Herstellung der substituierten Alkene der Formel (II) innerhalb eines bestimmten Bereiches variiert werden. Im allgemeinen arbeitet man bei Temperaturen zwischen -20°C und +100°C, vorzugsweise zwischen 0°C und 80°C.

Bei der obigen Umsetzung zur Herstellung der substituierten Alkene der Formel (II) setzt man die Reaktionskomponenten im allgemeinen in annähernd äquimolaren Mengen ein. Die Grignard-Verbindung der Formel (VI) wird jeweils frisch hergestellt. Die Aufarbeitung erfolgt nach den für derartige Grignard-Reaktionen üblichen Methoden.

Als Reaktionskomponenten kommen bei dem erfindungsgemäßen Verfahren (a) alle üblichen Reagenzien in Frage, die zur Epoxidierung geeignet sind. Bevorzugt verwendbar sind Persäuren, wie 3-Chlorperbenzoesäure, Peressigsäure und Perpropionsäure, ferner Wasserstoffperoxid, Sauerstoff und Peroxide, wie Natriumperoxid.

Diese Epoxidierungsmittel sind bekannte Stoffe.

Als Verdünnungsmittel kommen bei der Umsetzung nach dem erfindungsgemäßen Verfahren (a) alle für derartige Epoxidierungen üblichen inerten organischen Solventien in Betracht. Vorzugsweise verwendbar sind chlorierte Kohlenwasserstoffe, wie Methylenchlorid, Chloroform und Tetrachlorkohlenstoff.

Die Reaktionstemperaturen können bei der Durchführung des erfindungsgemäßen Verfahrens (a) innerhalb eines größeren Bereiches variiert werden. Im allgemeinen arbeitet man bei Temperaturen zwischen 0°C und 90°C, vorzugsweise zwischen 20°C und 80°C.

Das erfindungsgemäße Verfahren (a) wird im allgemeinen unter Normaldruck durchgeführt.

Bei der Durchführung des erfindungsgemäßen Verfahrens (a) setzt man auf 1 Mol an substituiertem Alken der Formel (II) im allgemeinen 1 bis 2 Mol an Epoxidierungsmittel ein. Die Aufarbeitung erfolgt nach üblichen Methoden. Im allgemeinen geht man so vor, daß man das Reaktionsgemisch filtriert, die organische Phase nacheinander mit wäßrigalkalischer Lösung und mit Wasser wäscht, dann trocknet

und einengt. Das verbleibende Produkt kann durch Destillation, Umkristallisation oder auch durch Verrühren mit geeigneten Solventien gereinigt werden.

Die bei der Durchführung des erfindungsgemäßen Verfahrens (b) als Ausgangsstoffe benötigten substituierten Alkene der Formel (II) wurden bereits im Zusammenhang mit der Beschreibung des Verfahrens (a) im einzelnen behandelt. Die bei dem erfindungsgemäßen Verfahren (b) weiterhin als Ausgangsstoffe benötigten Verbindungen sind durch die Formel (III) allgemein definiert. In dieser Formel steht R mit Ausnahme von Wasserstoff vorzugsweise für alle diejenigen Bedeutungen, die für den Rest $R^1$ bereits im Zusammenhang mit der Beschreibung der erfindungsgemäßen Stoffe der Formel (I) als bevorzugt genannt wurden. Z steht vorzugsweise für Chlor, Brom, Jod, Tosylat ($CH_3$- 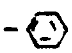 $-SO_3-$) und Mesylat ($CH_3SO_3-$).

Die Verbindungen der Formel (III) sind allgemein bekannte Stoffe der organischen Chemie.

In der zweiten Stufe des erfindungsgemäßen Verfahrens (b) kommen als Epoxidierungsmittel vorzugsweise alle diejenigen Stoffe in Betracht, die bereits im Zusammenhang mit der Beschreibung des erfindungsgemäßen Verfahrens (a) als Epoxidierungsmittel vorzugsweise genannt wurden.

Die bei dem erfindungsgemäßen Verfahren (b) als Zwischenprodukte anfallenden Verbindungen der Formel (IV) sind neu.

Als Säurebindemittel kommen bei der Durchführung der ersten Stufe des erfindungsgemäßen Verfahrens (b) alle üblichen Säureakzeptoren in Frage. Vorzugsweise verwendbar sind Alkalihydroxide, wie Natriumhydroxid und Kaliumhydroxid, ferner Alkalihydride, wie Natriumhydrid, und außerdem tertiäre aliphatische oder aromatische Amine, wie zum Beispiel Pyridin.

Als Verdünnungsmittel kommen bei der Durchführung der ersten Stufe des erfindungsgemäßen Verfahrens (b) alle üblichen inerten organischen Solventien in Betracht. Vorzugsweise verwendbar sind Ether, wie Diethylether, Dioxan und Tetrahydrofuran und polare Lösungsmittel, wie Dimethylformamid, Acetonitril und Dimethylsulfoxid.

Die Reaktionstemperaturen können bei der Durchführung der ersten Stufe des erfindungsgemäßen Verfahrens (b) innerhalb eines größeren Bereiches variiert werden. Im allgemeinen arbeitet man bei Temperaturen zwischen -10°C und +100°C, vorzugsweise zwischen 10°C und 80°C.

Das erfindungsgemäße Verfahren (b) wird sowohl in der ersten als auch in der zweiten Stufe im allgemeinen unter Normaldruck durchgeführt.

Bei der Durchführung der ersten Stufe des erfindungsgemäßen Verfahrens (b) setzt man auf 1 Mol an substituiertem Alken der Formel (II) im allgemeinen 1 bis 2 Mol an einer Verbindung der Formel (III) sowie gegebenenfalls

1 bis 2 Mol an Säurebindemittel ein. Zweckmäßigerweise werden die Verbindüng der Formel (III) und das Säurebindemittel jeweils in äquivalenten Mengen eingesetzt. Die Aufarbeitung erfolgt nach üblichen Methoden. Im allgemeinen geht man so vor, daß man das Reaktionsgemisch einengt, den Rückstand mit einem organischen Lösungsmittel und mit Wasser versetzt, die organische Phase abtrennt, die wäßrige Phase mehrfach mit einem in Wasser wenig löslichen organischen Solvens extrahiert, die vereinigten organischen Phasen wäscht, trocknet und einengt. Die dabei verbleibenden Verbindungen der Formel (IV) können gegebenenfalls durch Destillation oder Umkristallisation gereinigt werden. Sie lassen sich direkt für die in der zweiten Stufe durchzuführende Epoxidierung verwenden. Diese zweite Stufe des erfindungsgemäßen Verfahrens (b) wird in gleicher Weise durchgeführt wie das erfindungsgemäße Verfahren (a).

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall

sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren

Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im

Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Pyrienlaria und Apfelschorf eingesetzt werden.

Besonders hervorzuheben ist, daß die erfindungsgemäßen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Im übrigen besitzen die erfindungsgemäßen Wirkstoffe auch insektizide, akarizide und herbizide Eigenschaften.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck

stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus

organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen

vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenenheiten richtet.

Auch beim Einsatz der erfindungsgemäßen Stoffe als Fungizide kann die Aufwandmenge je nach Art

0154806

der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,001 bis 0,02 %, am Wirkungsort erforderlich.

Bei einer Anwendung als Herbizide können die erfindungsgemäßen Wirkstoffe sowohl vor als auch nach dem Auflaufen der Pflanzen appliziert werden. Sie können auch vor der Saat in den Boden eingearbeitet werden.

Die angewandte Wirkstoffmenge kann beim Einsatz zur Unkrautbekämpfung in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

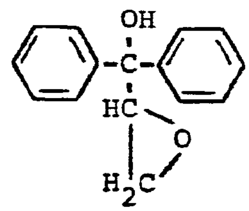

(I-1)

Ein Gemisch aus 70 g (0,333 Mol) 1,1-Diphenyl-prop-2-en-1-ol und 27 g (0,133 Mol) 3-Chlorperbenzoesäure (85 %ig) in 1000 ml Methylenchlorid wurde auf Rückflußtemperatur erhitzt. Nach 2 Stunden und nach 4 Stunden wurden jeweils 20 g (0,100 Mol) 3-Chlorperbenzoesäure hinzugefügt, und es wurde weitere 10 Stunden unter Rückfluß erhitzt. Danach wurde aufgearbeitet, indem man das abgekühlte Reaktionsgemisch filtrierte, die organische Phase nacheinander zweimal mit 5 %iger wäßriger Natriumcarbonat-Lösung und zweimal mit Wasser wusch. Die organische Phase wurde nach dem Trocknen über Natriumsulfat unter vermindertem Druck eingeengt. Der dabei verbleibende Rückstand wurde mit Wasser verrührt, abgesaugt und getrocknet. Man erhielt auf diese Weise 72,7 g (96,5 % der Theorie) an 2-(Diphenyl-hydroxymethyl)-oxiran in Form eines Festproduktes vom Schmelzpunkt 120-123°C.

Herstellung des Ausgangsproduktes:

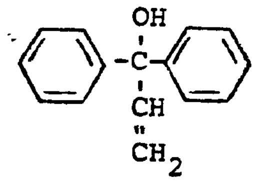

(II-1)

In ein Gemisch aus 34 g (1,4 Mol) Magnesiumspäne in 500 ml siedendem Tetrahydrofuran wurde eine Lösung von 150 g (1,4 Mol) Vinylbromid in 150 ml Tetrahydrofuran eingetropft. Nach beendeter Zugabe wurde noch 30 Minuten unter Rückfluß erhitzt. Anschließend wurde bei 40°C eine Lösung von 145 g (0,8 Mol) Benzophenon in 500 ml Tetrahydrofuran zugetropft. Das Reaktionsgemisch wurde noch 1 Stunde unter Rückfluß erhitzt, dann auf 0°C abgekühlt und auf 1 kg Eis gegossen. Die organische Phase wurde abgetrennt, und die wäßrige Phase wurde zweimal mit Diethylether extrahiert. Die vereinigten organischen Phasen wurden noch zweimal mit Wasser gewaschen, über Natriumsulfat getrocknet und unter vermindertem Druck eingeengt. Das verbleibende ölige Produkt erstarrte allmählich. Man erhielt auf diese Weise 165,8 g (79 % der Theorie) an 1,1-Diphenyl-prop-2-en-1-ol in Form eines Festproduktes vom Schmelzpunkt 96-98°C.

Beispiel 2

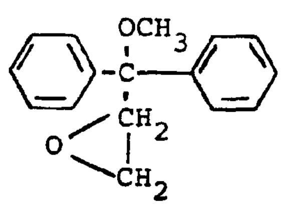

(I-2)

a) Herstellung der Verbindung der Formel

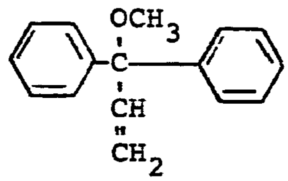

(IV-1)

Eine Mischung aus 75 g (0,357 Mol) 1,1-Diphenyl-prop-2-en-1-ol und 102 g (0,718 Mol) Methyliodid wurde bei Raumtemperatur unter Rühren in ein Gemisch aus 21,6 g (0,72 Mol) Natriumhydrid (80 %ig) in 100 ml Dimethylformamid eingetropft. Man rührte noch 2 Stunden bei Raumtemperatur und dann 4 Stunden bei 40°C. Danach wurde das Reaktionsgemisch unter vermindertem Druck eingeengt und der verbleibende Rückstand mit Diethylether und Wasser versetzt. Nach dem Abtrennen der organischen Phase wurde die wäßrige Phase noch einmal mit Diethylether extrahiert. Die vereinigten organischen Phasen wurden zweimal mit Wasser gewaschen, dann über Natriumsulfat getrocknet und unter vermindertem Druck eingeengt. Der verbleibende Rückstand wurde destilliert. Man erhielt auf diese Weise 70 g (87,5 % der Theorie) an 1,1-Diphenyl-1-methoxy-propen-2 in Form einer Flüssigkeit vom Siedepunkt 91°C/0,12 mbar.

b) Herstellung der Verbindung der Formel

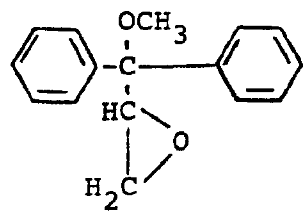

(I-2)

Ein Gemisch aus 70 g (0,312 Mol) 1,1-Diphenyl-1-methoxy-propen-2 und 23 g (0,114 Mol) 3-Chlorperbenzoesäure (85 %ig) in 1000 ml Methylenchlorid wurde auf Rückflußtemperatur erhitzt. Nach 2 Stunden und nach 4 Stunden wurden jeweils 20 g (0,100 Mol) 3-Chlorperbenzoesäure hinzugefügt, und es wurde weitere 12 Stunden unter Rückfluß erhitzt. Anschließend wurde aufgearbeitet, indem man das abgekühlte Reaktionsgemisch filtrierte, die organische Phase nacheinander zweimal mit 5 %iger wäßriger Natriumcarbonat-Lösung und zweimal mit Wasser wusch. Die organische Phase wurde nach dem Trocknen über Natriumsulfat unter vermindertem Druck eingeengt. Der verbleibende Rückstand wurde unter vermindertem Druck destilliert. Man erhielt auf diese Weise 70,5 g (93,3 % der Theorie) an 2-(1,1-Diphenyl-methoxymethyl)-oxiran in Form einer Flüssigkeit vom Siedepunkt 110-113°C/0,5 mbar.

Nach der im Beispiel 1 angegebenen Methode wurden auch die in der folgenden Tabelle 2 formelmäßig aufgeführten Verbindungen der Formel (I) hergestellt.

Tabelle 2

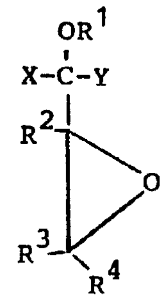

(I)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 3 | 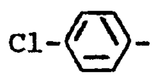 | 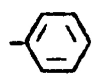 | H | H | H | H | 78 |
| 4 | 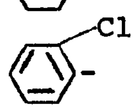 | 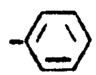 | H | H | H | H | 108 |
| 5 | 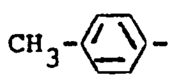 | 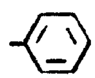 | H | H | H | H | 82-85 |
| 6 | 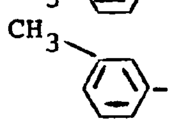 | 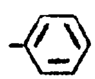 | H | H | H | H | 69 |
| 7 | 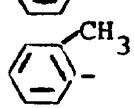 | 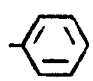 | H | H | H | H | 87-92 |
| 8 | 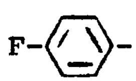 | 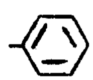 | H | H | H | H | 105 |
| 9 | 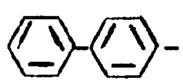 | 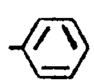 | H | H | H | H | 174 *) |

*) diastereomer zu Verbindung gemäß Bsp. Nr. 10

Tabelle 2 (Fortsetzung)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 10 | 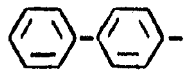 | 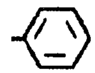 | H | H | H | H | 124-126 *) |
| 11 | 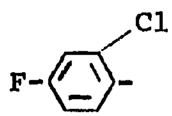 | 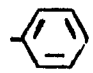 | H | H | H | H | 72- 75 |
| 12 | 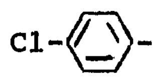 | 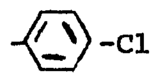 | H | H | H | H | 82- 83 |
| 13 | 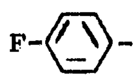 | 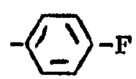 | H | H | H | H | 137 |
| 14 | 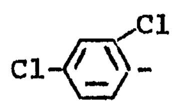 | H | H | H | H | H | Öl |
| 15 | 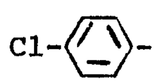 | $-CH_3$ | H | H | H | H | 53-56 |
| 16 | 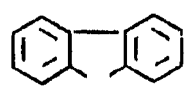 | | H | H | H | H | Öl |

*) diastereomer zu Verbindung gemäß Bsp. Nr. 9

Tabelle 2 (Fortsetzung)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 17 | $(CH_3)_3C-$ | $-CH_3$ | H | H | H | H | |
| 18 | 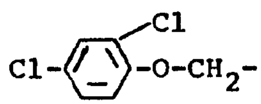 | $-C(CH_3)_3$ | H | H | H | H | Öl |
| 19 | 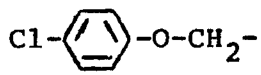 | $-C(CH_3)_3$ | H | H | H | H | Öl |
| 20 | 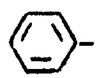 | $CH_3$ | H | H | H | H | 101-6/0,2 |
| 21 | 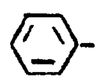 | 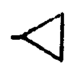 | H | H | H | H | 99/0,15 |
| 22 | 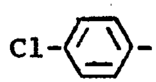 | H | H | H | H | H | Öl |
| 23 | 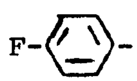 | $CH_3$ | H | H | H | H | Öl |
| 24 | 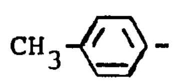 | $CH_3$ | H | H | H | H | Öl |

Tabelle 2 (Fortsetzung)

| Beispiel Nr. | X | Y | $R^1$ | $R^2$ | $R^3$ | $R^4$ | Schmelzpunkt /°C7 bzw. Siedepunkt /°C/mbar7 |
|---|---|---|---|---|---|---|---|
| 25 | 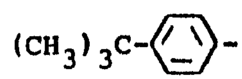 | $CH_3$ | H | H | H | H | 86 |
| 26 | 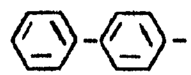 | $CH_3$ | H | H | H | H | 59-62 |
| 27 | 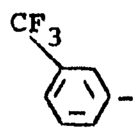 | $CH_3$ | H | H | H | H | Öl |
| 28 | 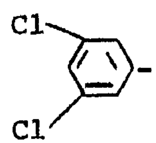 | $CH_3$ | H | H | H | H | 146/0,15 |
| 29 | 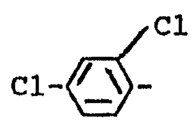 | $CH_3$ | H | H | H | H | 101-106/0,2 |

0154806



Tabelle 2 (Fortsetzung)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 30 | 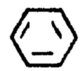 $-CH_2-$ | $CH_3$ | H | H | H | H | 73/0,1 |
| 31 | Cl-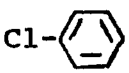- | 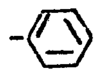 | H | H | $CH_3$ | $CH_3$ | 116 |
| 32 | Cl-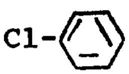- | 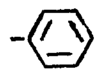 | H | $CH_3$ | H | H | 78-84 |
| 33 | Cl-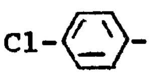- | 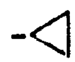 | H | H | H | H | Öl |
| 34 | Cl-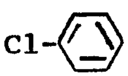- | $CH_3$ | H | H | $CH_3$ | $CH_3$ | 69 |
| 35 | Cl-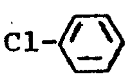- | $CH_3$ | H | $CH_3$ | H | H | 122-126/0,05 |
| 36 | Cl-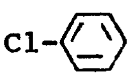$-CH_2-OCH_2-$ | $-C(CH_3)_3$ | H | H | H | H | 137-143/0,02 |
| 37 | 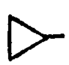 | 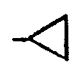 | H | H | H | H | 105/15 |

0154806

Nach der im Beispiel 2 angegebenen Methode wurden auch die in der folgenden Tabelle 3 formelmäßig aufgeführten Verbindungen der Formel (I) hergestellt.

Tabelle 3

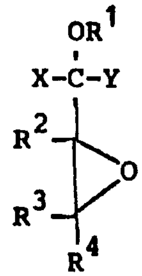

(I)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 38 | 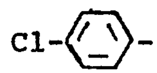 | 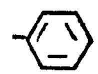 | $CH_3$ | H | H | H | Öl |
| 39 | 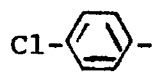 | 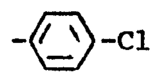 | $CH_3$ | H | H | H | 190-200/0,2 |
| 40 | 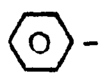 | 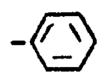 | 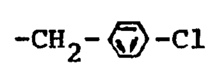 | H | H | H | Öl |
| 41 | 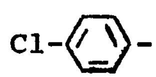 | 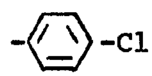 | $C_2H_5$ | H | H | H | 190/0,2 |
| 42 | 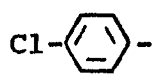 | 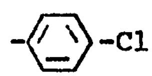 | $-(CH_2)_3-CH_3$ | H | H | H | 180/0,2 |
| 43 | 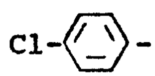 | $CH_3$ | $CH_3$ | H | H | H | 120/0,15 |

**Tabelle 3** (Fortsetzung)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 44 | 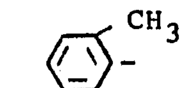 | 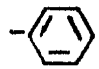 | $CH_3$ | H | H | H | Öl |
| 45 | 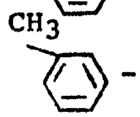 | 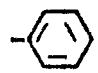 | $CH_3$ | H | H | H | Öl |
| 46 | 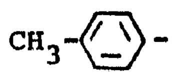 | 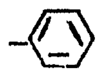 | $CH_3$ | H | H | H | Öl |
| 47 | 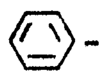 | $CH_3$ | $CH_3$ | H | H | H | Öl |
| 48 | 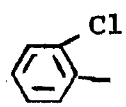 | 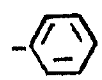 | $CH_3$ | H | H | H | Öl |
| 49 | 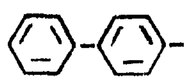 | $CH_3$ | $CH_3$ | H | H | H | Öl |
| 50 | 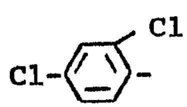 | $CH_3$ | $CH_3$ | H | H | H | 136-143/0,1 |

Tabelle 3 (Fortsetzung)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 51 | 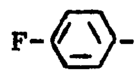 | $CH_3$ | $CH_3$ | H | H | H | 80-82/0,1 |
| 52 | 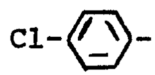 | H | $CH_3$ | H | H | H | 97/0,15 |
| 53 | 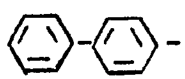 | 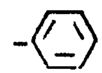 | $CH_3$ | H | H | H | Öl |
| 54 | 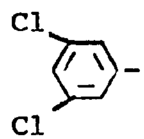 | $CH_3$ | $CH_3$ | H | H | H | 79/0,05 |
| 55 | 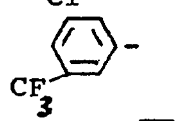 | $CH_3$ | $CH_3$ | H | H | H | Öl |
| 56 | 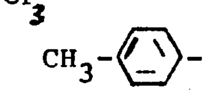 | $CH_3$ | $CH_3$ | H | H | H | Öl |
| 57 | $(CH_3)_2CH-$ | $CH_3$ | $CH_3$ | H | H | H | Öl |
| 58 | $-(CH_2)_4-$ | | $CH_3$ | H | H | H | Öl |

Nach der im Beispiel 1 angegebenen Methode wurden auch die in der folgenden Tabelle 4 formelmäßig aufgeführten Verbindungen der Formel (I) hergestellt. stellt.

**Tabelle 4**

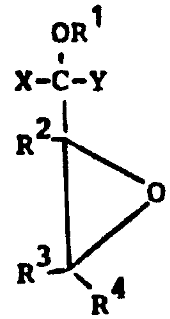

(I)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 59 | 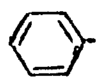 | 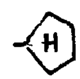 | H | H | H | H | öl |
| 60 | 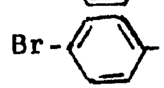 | $CH_3$ | H | H | H | H | öl |
| 61 | 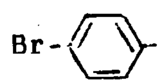 | 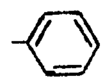 | H | H | H | H | 78 |
| 62 | 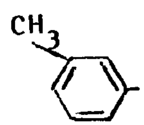 | $CH_3$ | H | H | H | H | öl |
| 63 | 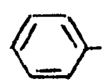 | $C_2H_5$- | H | H | H | H | öl |
| 64 | 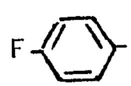 | $C_2H_5$- | H | H | H | H | 71 |

Tabelle 4 (Fortsetzung)

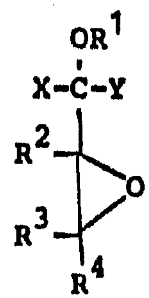

(I)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 65 | 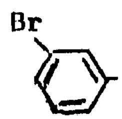 | $CH_3$ | H | H | H | H | öl |
| 66 | 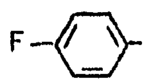 | 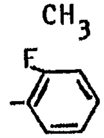 | H | H | H | H | 88 |
| 67 | 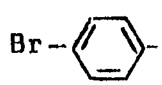 | 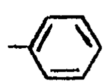 | H | H | H | H | öl |
| 68 | 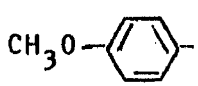 | $CH_3$ | H | H | H | H | 120/0,1 |
| 69 | 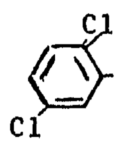 | $CH_3$ | H | H | H | H | 49 |
| 70 | 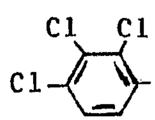 | $CH_3$ | H | H | H | H | öl |

**Tabelle 4** (Fortsetzung)

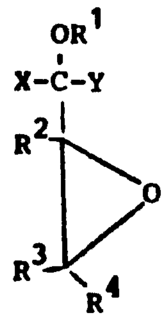

(I)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 71 | 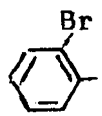 | $CH_3$ | H | H | H | H | öl |
| 72 | 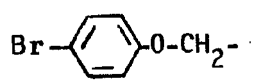 | 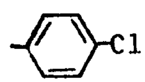 | H | H | H | H | öl |
| 73 | 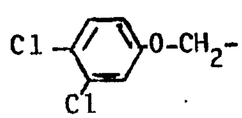 | 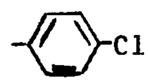 | H | H | H | H | öl |
| 74 | 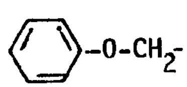 | 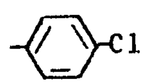 | H | H | H | H | 172/0,1 |
| 75 | 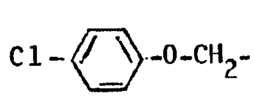 | 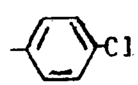 | H | H | H | H | öl |
| 76 | 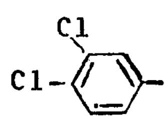 | $CH_3$ | H | H | H | H | öl |

**Tabelle 4** (Fortsetzung)

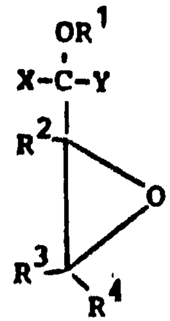

(I)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 77 | 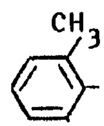 | $CH_3$ | H | H | H | H | Öl |

Nach der im Beispiel 2 angegebenen Methode wurden auch die in der folgenden Tabelle 5 formelmäßig aufgeführten Verbindungen der Formel (I) hergestellt.

Tabelle 5

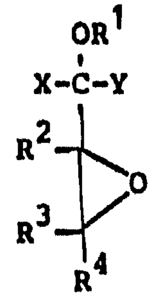 (I)

| Beispiel Nr. | X | Y | $R^1$ | R | $R^3$ | $R^4$ | Schmelzpunkt /°C/ bzw. Siedepunkt /°C/mbar/ |
|---|---|---|---|---|---|---|---|
| 78 | 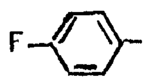 | 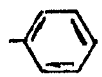 | $CH_3$ | H | H | H | öl |
| 79 | 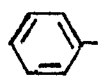 | 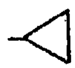 | $CH_3$ | H | H | H | öl |
| 80 | 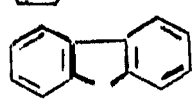 | | $CH_3$ | H | H | H | 79 |
| 81 | 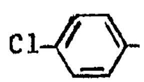 | 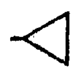 | $CH_3$ | H | H | H | öl |
| 82 | 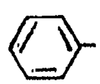 | $CH_3$ | $-CH_2-$ 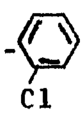 | H | H | H | öl |
| 83 | 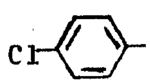 | $CH_3$ | $-CH_2-$ 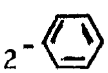 | H | H | H | 142/0,04 |
| 84 | 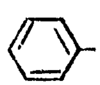 | 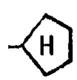 | $CH_3$ | H | H | H | öl |

**Tabelle 5** (Fortsetzung)

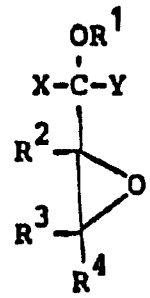

(I)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 85 | 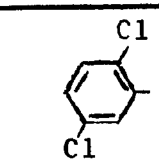 | $CH_3$ | $CH_3$ | H | H | H | Öl |
| 86 | 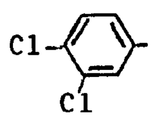 | $CH_3$ | $CH_3$ | H | H | H | Öl |
| 87 | 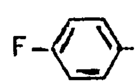 | 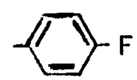 | $CH_3$ | H | H | H | Öl |
| 88 | 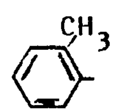 | $CH_3$ | $CH_3$ | H | H | H | Öl |
| 89 | 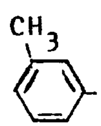 | $CH_3$ | $CH_3$ | H | H | H | Öl |

Tabelle 5 (Fortsetzung)

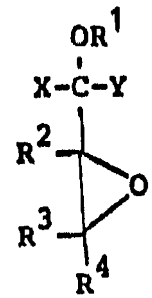

(I)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 90 | 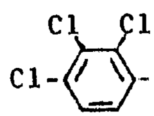 | $CH_3$ | $CH_3$ | H | H | H | öl |
| 91 | 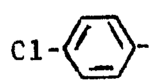 | $CH_3$ | 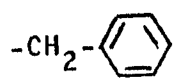 | H | H | H | 142/0,04 |
| 92 | 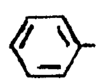 | $C_2H_5$ | $CH_3$ | H | H | H | 82-86/0,1 |
| 93 | 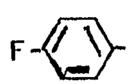 | $C_2H_5$ | $CH_3$ | H | H | H | 87/0,1 |
| 94 | 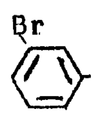 | $CH_3$ | $CH_3$ | H | H | H | 107/0,1 |
| 95 | 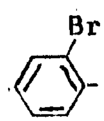 | $CH_3$ | $CH_3$ | H | H | H | 103/0,1 |

Tabelle 5 (Fortsetzung)

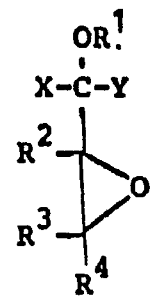

(I)

| Beispiel Nr. | X | Y | $R^1$ | $R^2$ | $R^3$ | $R^4$ | Schmelzpunkt [°C] bzw. Siedepunkt [°C/mbar] |
|---|---|---|---|---|---|---|---|
| 96 | 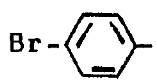 | $CH_3$ | $CH_3$ | H | H | H | 110/0,1 |

Nach der im Beispiel 1 angegebenen Methode wurden auch die in der folgenden Tabelle 6 formelmäßig aufgeführten Ausgangsverbindungen der Formel (II) hergestellt.

Tabelle 6

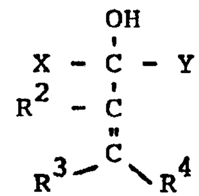

(II)

| Beispiel Nr. | X | Y | $R^2$ | $R^3$ | $R^4$ | Schmelzpunkt [°C] bzw. Siedepunkt [°C/mbar] |
|---|---|---|---|---|---|---|
| 97 | 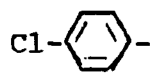 | 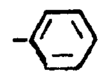 | H | H | H | Öl |
| 98 | 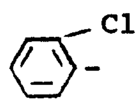 | 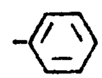 | H | H | H | Öl |
| 99 | 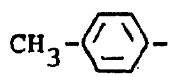 | 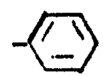 | H | H | H | Öl |
| 100 | 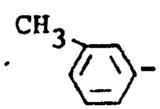 | 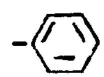 | H | H | H | Öl |
| 101 | 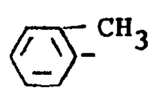 | 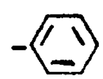 | H | H | H | Öl |
| 102 | 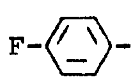 | 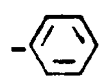 | H | H | H | Öl |

Tabelle 6 (Fortsetzung)

| Beispiel Nr. | X | Y | $R^2$ | $R^3$ | $R^4$ | Schmelzpunkt /°C/ bzw. Siedepunkt /°C/mbar/ |
|---|---|---|---|---|---|---|
| 103 | 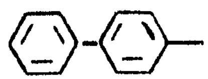 | 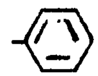 | H | H | H | 71-75 |
| 104 | 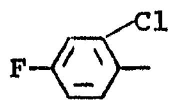 | 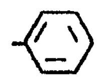 | H | H | H | Öl |
| 105 | 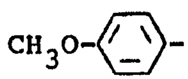 | 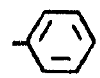 | H | H | H | Öl |
| 106 | 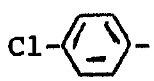 | 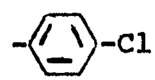 | H | H | H | Öl |
| 107 | 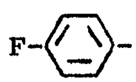 | 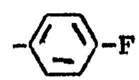 | H | H | H | Öl |
| 108 | 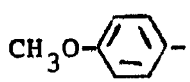 | 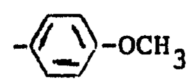 | H | H | H | Öl |
| 109 | 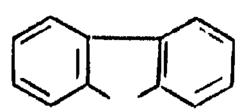 | | H | H | H | 86-88 |
| 110 | 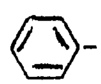 | $CH_3$ | H | H | H | Öl |

Tabelle 6 (Fortsetzung)

| Beispiel Nr. | X | Y | $R^2$ | $R^3$ | $R^4$ | Schmelzpunkt /°C/ bzw. Siedepunkt /°C/mbar/ |
|---|---|---|---|---|---|---|
| 111 | 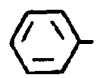 | 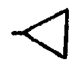 | H | H | H | öl |
| 112 | 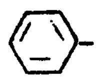 | 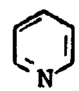 | H | H | H | öl |
| 113 | 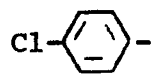 | H | H | H | H | öl |
| 114 | 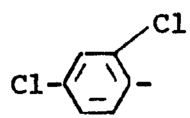 | H | H | H | H | öl |
| 115 | 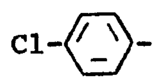 | $CH_3$ | H | H | H | öl |
| 116 | 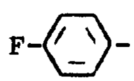 | $CH_3$ | H | H | H | öl |
| 117 | 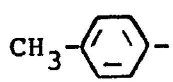 | $CH_3$ | H | H | H | öl |
| 118 | 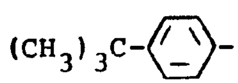 | $CH_3$ | H | H | H | öl |

Tabelle 6 (Fortsetzung)

| Beispiel Nr. | X | Y | $R^2$ | $R^3$ | $R^4$ | Schmelzpunkt [°C] bzw. Siedepunkt [°C/mbar] |
|---|---|---|---|---|---|---|
| 119 | 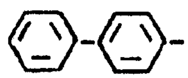 | $CH_3$ | H | H | H | Öl |
| 120 | 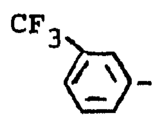 | $CH_3$ | H | H | H | Öl |
| 121 | 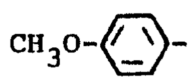 | 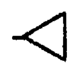 | H | H | H | Öl |
| 122 | 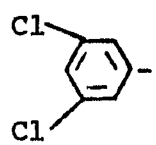 | $CH_3$ | H | H | H | Öl |
| 123 | 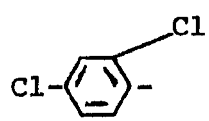 | $CH_3$ | H | H | H | Öl |
| 124 | 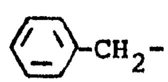 | $CH_3$ | H | H | H | |

Tabelle 6 (Fortsetzung)

| Beispiel Nr. | X | Y | $R^2$ | $R^3$ | $R^4$ | Schmelzpunkt /°C/ bzw. Siedepunkt /°C/mbar/ |
|---|---|---|---|---|---|---|
| 125 | $(CH_3)_2CH-$ | $CH_3$ | H | H | H | |
| 126 | $-(CH_2)_4-$ | | H | H | H | |
| 127 | $(CH_3)_3C-$ | $CH_3$ | H | H | H | |
| 128 | 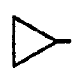 | 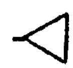 | H | H | H | |
| 129 | 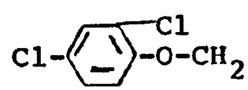 | $-C(CH_3)_3$ | H | H | H | |
| 130 | 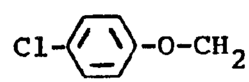 | $-C(CH_3)_3$ | H | H | H | |

Nach der im Beispiel 2 angegebenen Methode wurden auch die in der folgenden Tabelle 7 formelmäßig aufgeführten Ausgangsverbindungen der Formel (IV) hergestellt.

**Tabelle 7**

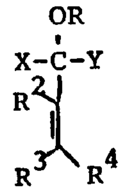

(IV)

| Beispiel Nr. | X | Y | R | $R^2$ | $R^3$ | $R^4$ | Schmelzpunkt /°C/ bzw. Siedepunkt /°C/mbar/ |
|---|---|---|---|---|---|---|---|
| 131 | 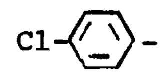 | 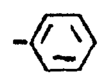 | $CH_3$ | H | H | H | Öl |
| 132 | 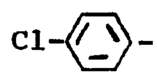 | 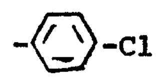 | $CH_3$ | H | H | H | 157-62/0,1 |
| 133 | 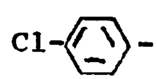 | 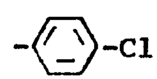 | $C_2H_5$ | H | H | H | 146/0,09 |
| 134 | 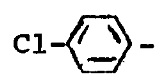 | 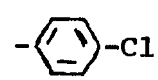 | $-(CH_2)_3CH_3$ | H | H | H | 152/0,1 |
| 135 | 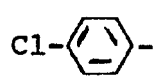 | $CH_3$ | $CH_3$ | H | H | H | 115-122/15 |

In den folgenden Verwendungsbeispielen wurden die nachstehend angegebenen Substanzen als Vergleichskomponenten eingesetzt.

(A) = 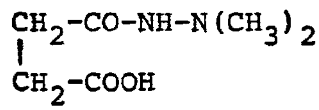

(Bernsteinsäure-mono-N-dimethylhydrazid)

(B) = 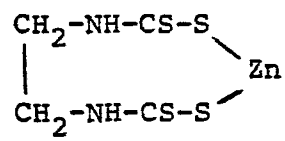

(Zink-ethylen-1,2-bis-dithiocarbamidat)

Beispiel A

Wuchshemmung bei Gerste

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

In diesem Test zeigen die in den Beispielen 3, 18, 38 und 39 aufgeführten erfindungsgemäßen Stoffe eine bessere Wirkung als die Vergleichssubstanz (A).

Beispiel B

Wuchshemmung bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

In diesem Test zeigen die in den Beispielen 10 und 38 aufgeführten erfindungsgemäßen Stoffe eine bessere Wirkung als die Vergleichssubstanz (A).

Beispiel C

Wuchshemmung bei Gras (Festuca pratensis)

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

In diesem Test zeigen die in den Beispielen 3, 9, 15, 38 und 39 aufgeführten erfindungsgemäßen Wirkstoffe eine bessere Wirkung als die Vergleichssubstanz (A).

Beispiel D

Pyricularia-Test (Reis) systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

In diesem Test zeigten die in den Beispielen 3, 14, 15, 18 und 38 aufgeführten erfindungsgemäßen Stoffe eine bessere Wirksamkeit als die Vergleichssubstanz (B).

Beispiel E

Pre-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigte der in dem Beispiel 8 aufgeführte Wirkstoff eine sehr gute Aktivität bei der selektiven Bekämpfung von Echinochloa, Digitaria und Setaria in Sojabohnen.

Patentansprüche

1. Substituierte Oxirane der Formel

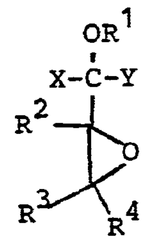

(I)

in welcher

X für gegebenenfalls substituiertes Alkyl, Cycloalkyl, gegebenenfalls substituiertes Phenyl, gegebenenfalls substituiertes Phenylalkyl, gegebenenfalls substituiertes Phenoxyalkyl oder gegebenenfalls substituiertes Phenalkoxyalkyl steht,

Y für Wasserstoff, gegebenenfalls substituiertes Alkyl, gegebenenfalls substituiertes Aryl, gegebenenfalls substituiertes Aralkyl, für gegebenenfalls substituiertes Cycloalkyl oder für Pyridyl steht, oder

X und Y gemeinsam für eine Alkylenkette mit 4 bis 6 Kohlenstoffatomen oder für die Reste der Formeln

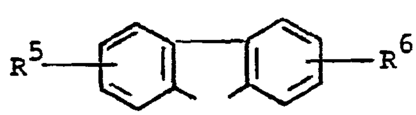

und

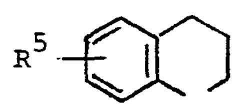

stehen, worin

$R^5$ und $R^6$ unabhängig voneinander für Wasserstofff, Halogen, Alkyl, Alkoxy, Alkylthio, Halogenalkyl oder Phenyl stehen,

$R^1$ für Wasserstoff, gegebenenfalls substituiertes Alkyl, Acyl, Alkenyl oder gegebenenfalls substituiertes Aralkyl steht und

$R^2$, $R^3$ und $R^4$ unabhängig voneinander für Wasserstoff oder Alkyl stehen.

2. Substituierte Oxirane der Formel (I), in welcher

X für gegebenenfalls durch Halogen, Alkoxy mit 1 bis 6 Kohlenstoffatomen und/oder Alkylthio mit 1 bis 6 Kohlenstoffatomen substituiertes Alkyl mit 1 bis 12 Kohlenstoffatomen, Cycloalkyl mit 3 bis 8 Kohlenstoffatomen, für den Rest der Formel

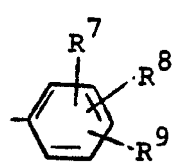

,

in welcher

$R^7$, $R^8$ und $R^9$ unabhängig voneinander für Wasserstoff, Halogen, Alkyl, Alkoxy, Alkylthio, Halogenalkyl oder Phenyl stehen, oder

X für gegebenenfalls durch Halogen und/oder Alkyl substituiertes Phenylalkyl mit 1 bis 4 Kohlenstoffatomen im Alkylteil, für gegebenenfalls durch Halogen und/oder Alkyl substituiertes Phenoxyalkyl mit 1 bis 4 Kohlenstoffatomen im Alkylteil oder für gegebenenfalls durch Halogen und/oder Alkyl substituiertes Phenalkoxyalkyl mit 1 bis 4 Kohlenstoffatomen in der Alkoxygruppe und 1 bis 4 Kohlenstoffatomen im Alkylteil steht,

Y für Wasserstoff, gegebenenfalls durch Halogen, Alkoxy mit 1 bis 6 Kohlenstoffatomen und/oder Alkylthio mit 1 bis 6 Kohlenstoffatomen substituiertes Alkyl mit 1 bis 12 Kohlenstoffatomen, gegebenenfalls durch Halogen, Alkyl, Alkoxy, Alkylthio, Halogenalkyl und/oder Phenyl substituiertes Aryl mit 6 bis 10 Kohlenstoffatomen, für gegebenenfalls durch Halogen, Alkyl, Alkoxy, Alkylthio, Halogenalkyl und/oder Phenyl substituiertes Aralkyl mit 6 bis 10 Kohlenstoffatomen im Arylteil und 1 bis 4 Kohlenstoffatomen im Alkylteil, für gegebenenfalls durch Alkyl mit 1 bis 6 Kohlenstoffatomen substituiertes Cycloalkyl mit 3 bis 8 Kohlenstoffatomen oder für Pyridyl steht, oder

X und Y gemeinsam für eine Alkylenkette mit 4 oder 5 Kohlenstoffatomen oder für die Reste der Formeln

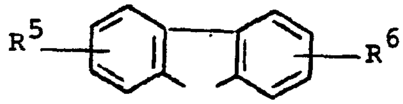

und

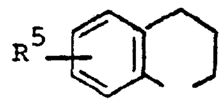

stehen, in welchen

$R^5$ und $R^6$ unabhängig voneinander für Wasserstoff, Fluor, Chlor, Brom, Alkyl mit 1 bis 6 Kohlenstoffatomen, Alkoxy mit 1 bis 6 Kohlenstoffatomen, Alkylthio mit 1 bis 6 Kohlenstoffatomen, Halogenalkyl mit 1 bis 6 Kohlenstoffatomen und 1 bis 5 Halogenatomen oder Phenyl stehen,

$R^1$ für Wasserstoff, gegebenenfalls durch Fluor, Chlor, Brom, Alkoxy mit 1 bis 6 Kohlenstoffatomen und/oder Alkylthio mit 1 bis 6 Kohlenstoffatomen substituiertes Alkyl mit 1 bis 6 Kohlenstoffatomen, Alkylcarbonyl mit 1 bis 6 Kohlenstoffatomen im Alkylteil, Phenylcarbonyl, Alkenyl mit 2 bis 8 Kohlenstoffatomen, oder für gegebenenfalls durch Halogen und/oder Alkyl substituiertes Aralkyl mit 6 bis 10 Kohlenstoffatomen im Arylteil und 1 bis 4 Kohlenstoffatomen im Alkylteil steht und

$R^2$, $R^3$ und $R^4$ unabhängig voneinander für Wasserstoff oder Alkyl mit 1 bis 4 Kohlenstoffatomen stehen.

3. Verfahren zur Herstellung von substituierten Oxiranen der Formel

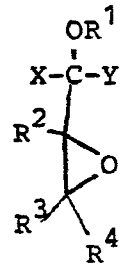

(I)

in welcher

X für gegebenenfalls substituiertes Alkyl, Cycloalkyl, gegebenenfalls substituiertes Phenyl, gegebenenfalls substituietes Phenylalkyl, gegebenenfalls substituiertes Phenoxyalkyl oder gegebenenfalls substituiertes Phenalkoxyalkyl steht,

Y für Wasserstoff, gegebenenfalls substituiertes Alkyl, gegebenenfalls substituiertes Aryl, gegebenenfalls substituiertes Aralkyl, für gegebenenfalls substituiertes Cycloalkyl oder für Pyridyl steht, oder

X und Y gemeinsam für eine Alkylenkette mit 4 bis 6 Kohlenstoffatomen oder für die Reste der Formeln

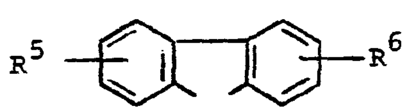

und

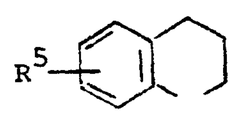

stehen, worin

$R^5$ und $R^6$ unabhängig voneinander für Wasserstoff, Halogen, Alkyl, Alkoxy, Alkylthio, Halogenalkyl oder Phenyl stehen,

$R^1$ für Wasserstoff, gegebenenfalls substituiertes Alkyl, Acyl, Alkenyl oder gegebenenfalls substituiertes Aralkyl steht und

$R^2$, $R^3$ und $R^4$ unabhängig voneinander für Wasserstoff oder Alkyl stehen,

dadurch gekennzeichnet, daß man

a) substituierte Alkene der Formel

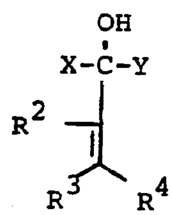

(II)

in welcher

X, Y, $R^2$, $R^3$ und $R^4$ die oben angegebene Bedeutung haben,

mit Epoxidierungsmitteln gegebenenfalls in Gegenwart eines inerten organischen Verdünnungsmittels umsetzt,

oder

b) substituierte Alkene der Formel

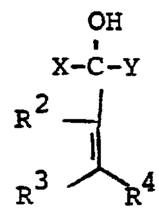

(II)

in welcher
X, Y, $R^2$, $R^3$ und $R^4$ die oben angegebene Bedeutung haben,

zunächst mit Verbindungen der Formel

$$Z - R \qquad (III)$$

in welcher

R für gegebenenfalls substituiertes Alkyl, Acyl, Alkenyl oder gegebenenfalls substituiertes Aralkyl steht und

Z für Halogen, Tosylat oder Mesylat steht,

gegebenenfalls in Gegenwart eines Säurebindemittels und gegebenenfalls in Gegenwart eines inerten organischen Verdünnungsmittels umsetzt und die dabei anfallenden Verbindungen der Formel

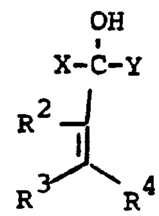

(IV)

in welcher
X, Y, R, $R^2$, $R^3$ und $R^4$ die oben angegebene Bedeutung haben,

dann mit Epoxidierungsmitteln gegebenenfalls in Gegenwart eines inerten organischen Verdünnungsmittels umsetzt.

4. Pflanzenbehandlungsmittel, gekennzeichnet durch einen Gehalt an mindestens einem substituierten Oxiran der Formel (I).

5. Verfahren zur Behandlung von Pflanzen, dadurch gekennzeichnet, daß man substituierte Oxirane der Formel (I) auf die Pflanzen und/oder deren Lebensraum ausbringt.

6. Verwendung von substituierten Oxiranen der Formel (I) zur Behandlung von Pflanzen.

7. Verfahren zur Herstellung von Pflanzenbehandlungsmitteln, dadurch gekennzeichnet, daß man substituierte Oxirane der Formel (I) mit Streckmitteln und/oder oberflächenaktiven Stoffen vermischt.

8. Substituierte Alkene der Formel

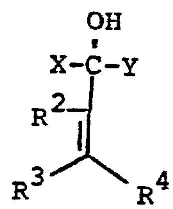

(II)

in welcher

X für gegebenenfalls substituiertes Alkyl, Cycloalkyl, gegebenenfalls substituiertes Phenyl, gegebenenfalls substituiertes Phenylalkyl, gegebenenfalls substituiertes Phenoxyalkyl oder gegebenenfalls substituiertes Phenalkoxyalkyl steht,

Y für Wasserstoff, gegebenenfalls substituiertes Alkyl, gegebenenfalls substituiertes Aryl, gegebenenfalls substituiertes Aralkyl, für gegebenenfalls substituiertes Cycloalkyl oder für Pyridyl steht,

oder

X und Y gemeinsam für eine Alkylenkette mit 4 bis 6 Kohlenstoffatomen oder für die Reste der Formeln

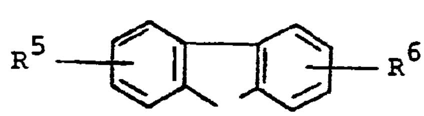 und 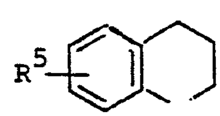

stehen, worin

$R^5$ und $R^6$ unabhängig voneinander für Wasserstoff, Halogen, Alkyl, Alkoxy, Alkylthio, Halogenalkyl oder Phenyl stehen, und

$R^2$, $R^3$ und $R^4$ unabhängig voneinander für Wasserstoff oder Alkyl stehen.

9. Verfahren zur Herstellung von substituierten Alkenen der Formel

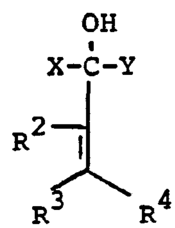

(II)

in welcher

X für gegebenenfalls substituiertes Alkyl, Cycloalkyl, gegebenenfalls substituiertes Phenyl, gegebenenfalls substituiertes Phenylalkyl, gegebenenfalls substituiertes Phenoxyalkyl oder gegebenenfalls substituiertes Phenalkoxyalkyl steht,

Y für Wasserstoff, gegebenenfalls substituiertes Alkyl, gegebenenfalls substituiertes Aryl, gegebenenfalls substituiertes Aralkyl, für gegebenenfalls substituiertes Cycloalkyl oder für Pyridyl steht,

oder

X und Y gemeinsam für eine Alkylenkette mit 4 bis 6

Kohlenstoffatomen oder für die Reste der Formeln

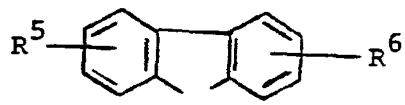 und 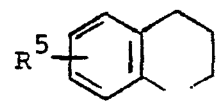

stehen, worin

$R^5$ und $R^6$ unabhängig voneinander für Wasserstoff, Halogen, Alkyl, Alkoxy, Alkylthio, Halogenalkyl oder Phenyl stehen,

und

$R^2$, $R^3$ und $R^4$ unabhängig voneinander für Wasserstoff oder Alkyl stehen,

dadurch gekennzeichnet, daß man Ketone oder Aldehyde der Formel

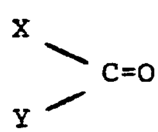 (V)

in welcher

X und Y die oben angegebene Bedeutung haben,

mit Grignard-Reagentien der Formel

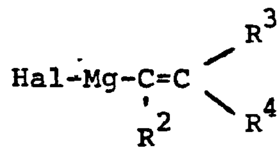

(VI)

in welcher

$R^2$, $R^3$ und $R^4$ die oben angegebene Bedeutung haben und

Hal für Brom oder Jod steht,

in Gegenwart eines inerten Verdünnungsmittels umsetzt.

10. Verbindungen der Formel

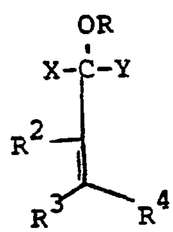

(IV)

in welcher

R für gegebenenfalls substituiertes Alkyl, Acyl, Alkenyl oder gegebenenfalls substituiertes Aralkyl steht,

X für gegebenenfalls substituiertes Alkyl, Cycloalkyl, gegebenenfalls substituiertes Phenyl, gegebenenfalls substituiertes Phenylalkyl, gegebenenfalls substituiertes Phenoxyalkyl oder gegebenenfalls substituiertes Phenalkoxyalkyl steht,

Y für Wasserstoff, gegebenenfalls substituiertes Alkyl, gegebenenfalls substituiertes Aryl, gegebenenfalls substituiertes Aralkyl, für gegebenenfalls substituiertes Cycloalkyl oder für Pyridyl steht,

oder

X und Y gemeinsam für eine Alkylenkette mit 4 bis 6 Kohlenstoffatomen oder für die Reste der Formeln

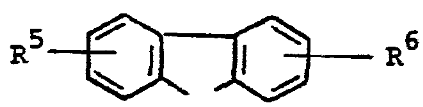 und 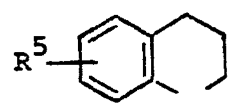

stehen, worin

$R^5$ und $R^6$ unabhängig voneinander für Wasserstoff, Halogen, Alkyl, Alkoxy, Alkylthio, Halogenalkyl oder Phenyl stehen,

und

$R^2$, $R^3$ und $R^4$ unabhängig voneinander für Wasserstoff oder Alkyl stehen.